Europäisches Patentamt
European Patent Office
Office européen des brevets

(19)

(11) Publication number: **0 123 319**
**A2**

(12)

# EUROPEAN PATENT APPLICATION

(21) Application number: 84104662.6

(22) Date of filing: 25.04.84

(51) Int. Cl.³: **C 07 D 249/08**
C 07 D 233/60, A 01 N 43/64
A 01 N 43/50

(30) Priority: 26.04.83 IT 2078083

(43) Date of publication of application:
31.10.84 Bulletin 84/44

(84) Designated Contracting States:
BE CH DE FR GB LI NL

(71) Applicant: Montedison S.p.A.
Patents & Licensing Dept. Foro Buonaparte, 31 P.O. Box 10528
I-20121 Milan(IT)

(72) Inventor: Colle, Roberto, Dr.
821, Residenza Parco
Milano 3, Basiglio Milan(IT)

(72) Inventor: Gozzo, Franco, Dr.
52 P, via Triulziana
San Donato Milanese Milan(IT)

(72) Inventor: Mirenna, Luigi
4, via Gamboloita
Milan(IT)

(74) Representative: Schmied-Kowarzik, Volker, Dr. et al,
Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8
D-8000 München 40(DE)

(54) **Fungicidal unsaturated 3-azolyl-1,4-diketones.**

(57) There are disclosed compounds of formula:

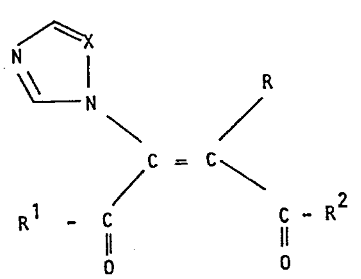

(I)

wherein X represents a nitrogen atom or a CH group;

R = alkyl, alkenyl, alkynyl, alkoxy, phenyl or phenoxy groups optionally substituted, alkoxycarbonyl, alkylcarbonyl, benzoyl or cyano groups;

R¹ and R² = alkyl, alkenyl, alkynyl groups, phenyl group optionally substituted, alkoxycarbonyl, alkylcarbonyl or cyano groups.

The compounds of formula I are endowed with fungicide activity and are useful for protecting agrarian cultivations against the action of phytopathogenous fungi.

EP 0 123 319 A2

Croydon Printing Company Ltd.

0123319

The present invention relates to unsaturated 1,4-diketones having fungicide activity and more precisely it relates to 1,4-diketones derivatives which are unsaturated between car= bon atoms 2 and 3 and are substituted on the carbon atom in position 3 by a heterocyclic radical derived from imidazole or from 1,2,4-triazole.

The invention relates also to the use of said compounds in the fight against fungus infections in useful plants and to the fungicide compositions containing these compounds as ac= tive ingredient.

The unsaturated 1,4-diketones, which form object of the pre= sent invention, have formula:

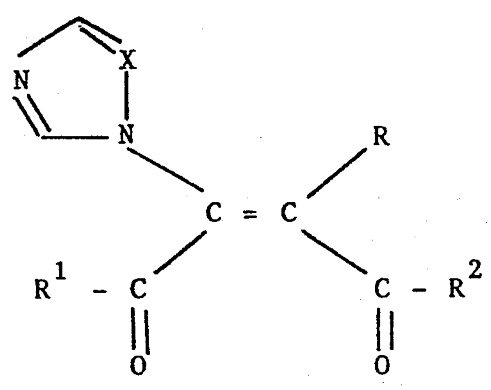

(I)

wherein:

X represents a nitrogen atom or a CH group;

R represents an alkyl group having from 1 to 4 carbon atoms; an alkenyl or an alkynyl group having from 2 to 5 carbon atoms; an alkoxy group having from 1 to 4 carbon atoms, a phenyl group or a phenoxy group optionally substituted

by one or more groups selected among halogen atoms, alkyl groups having from 1 to 4 carbon atoms, alkoxy groups ha=ving from 1 to 4 carbon atoms, nitro group; an alkoxycar=bonyl or an alkylcarbonyl group having from 1 to 4 carbon atoms in the alkylic part; a benzoyl or cyano group;

$R^1$ and $R^2$ (equal to or different from each other) represent an alkyl group having from 1 to 4 carbon atoms; an alkenyl or an alkynyl group having from 2 to 5 carbon atoms; a phe=nyl group optionally substituted by one or more groups se=lected among halogen atoms, alkyl groups having from 1 to 4 carbon atoms, alkoxy groups having from 1 to 4 carbon atoms, nitro group;

an alkoxy-carbonyl or an alkylcarbonyl group having from 1 to 4 carbon atoms in the alkylic part; a cyano group.

The compounds of formula I are endowed with high fungicide activity and with other useful properties, hereinafter descri=bed, which allow to make use of such compounds in agrarian field for protecting useful cultivations against the action of phytopathogenous fungi.

The preparation of the compounds of formula I is carried out by mild oxidation of the compounds of formula:

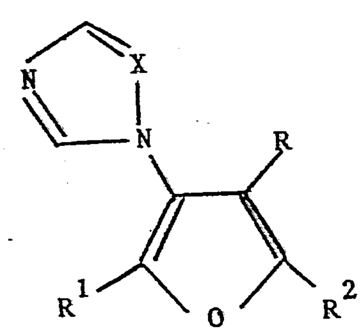

(II)

wherein R, $R^1$, $R^2$ and X have the same meanings recorded for formula I.

The compounds of formula II and the preparation thereof are described in copending EPC-application 84 103 557.9 in the name of the same Applicant.

The oxidation reaction of the compounds of formula II is car=ried out in a solution of glacial acetic acid in the presen=ce of an excess of concentrated nitric acid (at 65%) at tem=peratures ranging between 0 and 30°C.

Alternatively the oxidation reaction may be carried out in or=ganic acids, such as, for instance, glacial acetic acid in the presence of hydrogen peroxide at 30% at temperatures ran=ging between the room temperature and the reflux temperature of the mixture, or also by means of lead tetraacetate in ace=tic acid or chloroform generally at the reflux temperature of the reaction mixture.

As above mentioned the compounds of formula I are endowed with high fungicide activity.

They possess a wide action spectrum, as they are active against phytopathogenous fungi belonging to various genera of nume=rous families such as for instance Piricularia, Puccinia, Erysiphe, Sphaerotheca, Botrytis, Phytophtora, Venturia, Fu=sarium, Plasmopara, Peronospora, Pythium and so on.

Therefore the compounds of formula I are useful for fighting quite a number of plant deseases and they result particularly active against those deseases which are usually known as oidium and rust.

As antioidic compounds, the compounds according to the invention result to be endowed with a very high or complete activity even at extremely low doses.

Furthermore the compounds of formula I possess other positive characteristics such as a fungicide action having both preventive and curative characters and a complete compatibility with the plants to be protected against the fungus infection.

Owing to the high fungicide activity coupled with the above mentioned positive characteristics, the fungicide compounds, object of the present invention, may be used for protecting quite a number of useful cultivations from the fungus action; among these useful cultivations we can cite: vine, rice, Gramineae, tomato, tobacco and other solanaceae, horticultural cultivations, strawberries, cucurbitaceae, fruit trees and ornamental plants. They can be used for protecting foods stuff as well.

For the practical uses in agriculture it is often useful to have available fungicide compositions containing one or more

compounds of formula I as active ingredient.

Such compositions, which according to the normal formulative practice, are in the form of dry powders, wettable powders, emulsifiable concentrates, pastes, granular formulates, etc. consist of one or more compounds of formula I as active ingredient, of a solid or liquid carrier and optionally of other additives, such as, for instance, surfactants, wetting agents, dispersing agents, suspending agents, etc.

If desired, it is possible to add to the compositions, object of the invention other compatible active substances as well, such as other fungicides, herbicides, phytogrowth regulators, fertilizers and insecticides.

The dose of active substance to be used, varies according to different factors; among these factors we can cite the kind, the degree and the stadium of the fungus infection, the cultivation to be protected, the specific effectiveness of the considered compound of formula I, climatic and environmental factors.

Owing to the high fungicide activity of the compounds of formula I, it is generally sufficient to employ amounts of active substance ranging between 10 and 2000 g/ha, preferably between 100 and 1500 g/ha.

European patent No. 0002331 discloses imidazole and triazole compounds. In this respect the compounds of the present application possess superior fungicidal activity.

The following examples are given to better illustrate the invention.

EXAMPLE 1

Preparation of compound 1,2,4-triphenyl-3-(1-triazolyl)-2-buten-1,4-dione (compound No. 1).

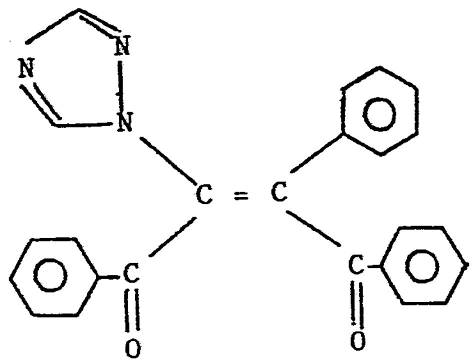

A solution containing 1 g of 2,4,5-triphenyl-3-(1-triazolyl)-furan /described in copending EPC-application 84 103 557.9/ in 20 ml of glacial acetic acid was added with 2 ml of $HNO_3$ at 65 %.

The mixture was kept for 30 minutes at room temperature.

Te solvent was then removed by evaporation at reduced pressure and the residue was diluted with water.

The insoluble solid was collected by filtration, washed with water and dried in the air.

By crystallization from ethanol 0.75 g of the desired product, were obtained; such a product was pure for thin-layer chroma=

tography (TLC) and was in the form of a crystalline solid (m.p. 168-170°C, IR was consistent with the assigned structure).

EXAMPLE 2

Preparation of compound 1-(4-chlorophenyl)-2(-1-triazolyl) -3-phenyl-5,5-dimethylhexen-1,4-dione (compound No. 2).

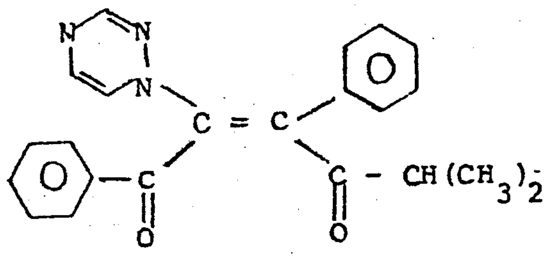

A solution containing 1 g of 2-(4-chlorophenyl)-4-phenyl-5-terbutyl-3-(1-triazolyl)-furano in 20 ml of glacial acid was added with 2 ml of $HNO_3$ at 65 %.

By proceeding as in Example 1, 0.70 g of the desired product were obtained; such a product was pure for thin layer chromatography (TCL) and was in the form of a crystalline solid (m.p. 163-165°C, IR consistent with the assigned structure).

EXAMPLE 3

Determination of the fungicide activity against cucumber oidium /Sphaerotheca fuliginea (Schlech) Salmon/.

Preventive activity:

Cucumber plants cv. Marketer, grown in pot in a conditioned environment, were sprayed on the lower leaf face with the product under examination in a water-acetone solution containing 20% of acetone (vol/vol.)

Then the plants were kept in a conditioned environment for 6 days and at the seventh day they were sprayed on the upper leaf face with an aqueous suspension of conidia of Sphaero=theca fuliginea (200.000 conidia/ml.). The plants were then carried back to the conditioned room.

At the end of the incubation period of the fungus (8 days), the infection degree was was evaluated by means of indexes of a scale of values from 100 (= sound plant) to 0 (= comple=tely infected plant).

Curative activity:

Cucumber plants cv. Marketer, grown in pot in conditioned environment, were sprayed on the upper leaf face with an aque=ous suspension of conidia of Sphaerotheca fuliginea (200.000 conidia/ml.) After 24 hours from the infection the plants were treated with the product under examination in a water-acetone solution containg 20% of acetone (vol./vol.), by sprin=kling both leaf faces. At the end of the incubation period of the fungus (8 days), during which time the plants were kept in a suitably conditioned environment, the infection degree was evaluated by means of indexes of a scale of va=lues from 100 (= sound plant) to 0 (= completely infected plant).

Compounds according to the invention exhibited a complete activity (100 % reduction of the fungus infection) at doses of 0.5 g/l in tests having both preventive and curative characters.

Furthermore, at lower doses as well, compounds according to the invention exhibited a high activity. For instance compound No 1 and 2 still exhibited 100 % of both curative and preventive activities, at a dose of 0.06 g/l.

In the above formulae the various residues have preferably the following definition.
Alkyl: methyl, ethyl, n-propyl, i-propyl, n-butyl, i-butyl, sec.-butyl, tert.-butyl.
In alkoxy groups or more complex groups containing an alkyl moiety (such as alkoxycarbonyl) alkyl is defined as above.
Alkenyl groups are vinyl, 1- or 2 propenyl, the butenyl and pentenyl groups.
Alkinyl groups are acetylenyl, 1 or 2 propynyl, the butynyl and pentynyl groups.
The phenyl or phenoxy group may be substituted by 1,2, 3 or 4 of the stated substituents.
Halogen is F, Cl, Br or J.

# CLAIMS



1) Compounds of formula:

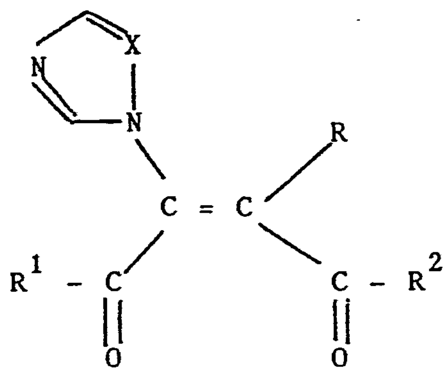

(I)

wherein:

X represents a nitrogen atom or a CH group;

R represents an alkyl group having from 1 to 4 carbon atoms; an alkenyl or an alkynyl group having from 2 to 5 carbon atoms; an alkoxy group having from 1 to 4 carbon atoms; a phenyl group or a phenoxy group optionally substituted by one or more groups selected among halogen atoms, alkyl groups having from 1 to 4 carbon atoms, alkoxy groups ha= ving from 1 to 4 carbon atoms, nitro group; an alkoxycarbonyl or an alkylcarbonyl group having from 1 to 4 carbon atoms in the alkylic part; a benzoyl or a cyano group;

$R^1$ and $R^2$ (equal to or different from each other) represent an alkyl group having from 1 to 4 carbon atoms; an alke= nyl or an alkynyl group having from 2 to 5 carbon atoms; a phenyl group optionally substituted by one or more groups selected among halogen atoms, alkyl groups having from 1 to 4 carbon atoms, alkoxy groups having from 1 to 4 car=

0123319

bon atoms, nitro group;

an alkoxycarbonyl or an alkylcarbonyl group having from 1 to 4 carbon atoms in the alkylic part; a cyano group.

2) Compounds according to claim 1, wherein X represents a nitrogen atom.

3) Compounds according to claim 2, wherein $R^1$ and $R^2$ repre= sent each of them an optionally substituted phenyl.

4) Compound 1,2,4-triphenyl-3-(1-triazolyl)-2-butene-1,4-dione.

5) Compound 1-(4-chlorophenyl)-2-(1-triazolyl)-3-phenyl-5,5-dimethylhexene-1,4-dione.

6) A process for preparing the compounds of claim 1, consisting in oxidizing under mild conditions a compound of formula:

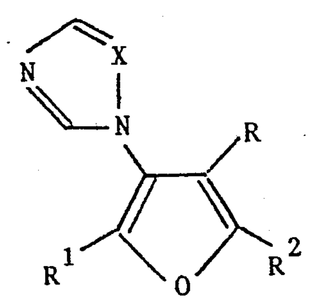

(II)

wherein X, R, $R^1$ and $R^2$ have the same meaning as in claim 1.

7) A process according to claim 6 characterized in that the oxidation of the compound of formula II is carried out in a solution of glacial acetic acid in the presence of an excess of concentrated nitric acid at a temperature ranging between 0 and 30°C.

8) A method for fighting fungus infections in useful plants consisting in distributing on the plants or in the area where they grow, when the fungus infection is foreseen or it is in progress already, an effective amount of a com= pound according to claim 1, as such or in the form of a suitable composition.

9) A method for fighting fungus infections in useful plants, according to claim 8, applied to the fight against the fungus infections known as oidium and rust.

10) A fungicide composition having as active ingredient one or more compounds according to claim 1, together with a solid or liquid carrier and optionally other additives.